Europäisches Patentamt

(19) European Patent Office  (11) Numéro de publication: **0 047 190**

Office européen des brevets  **B1**

(12)  **FASCICULE DE BREVET EUROPEEN**

(45) Date de publication du fascicule du brevet:  (51) Int. Cl.⁴: **A 61 F 9/00**
27.03.85

(21) Numéro de dépôt: 81400745.6

(22) Date de dépôt: 11.05.81

(54) Appareil chirurgical pour la découpe précise de la cornée.

(30) Priorité: 03.09.80 FR 8019014  (73) Titulaire: **LABORATOIRES HYDRON, 6, Villa E. Bergerat, F-92200 Neuilly-sur-Seine (FR)**

(43) Date de publication de la demande:
10.03.82 Bulletin 82/10  (72) Inventeur: **Hanna, Khalil, 5, rue Montmartre, F-75001 Paris (FR)**

(45) Mention de la délivrance du brevet:
27.03.85 Bulletin 85/13  (74) Mandataire: **Corre, Jacques Denis Paul et al, Cabinet Regimbeau 26, Avenue Kléber, F-75116 Paris (FR)**

(84) Etats contractants désignés:
**AT BE CH DE FR GB IT LI LU NL SE**

(56) Documents cités:
**DE - A - 2 811 869**
**FR - A - 1 106 449**
**FR - A - 2 364 646**
**US - A - 2 838 050**

## Description

La présente invention concerne un appareil chirurgical ou »trépan« pour la découpe précise de la cornée.

La kératoplastie nécessite l'ablation de la partie de cornée de l'oeil affectée pour la remplacer par une greffe, c'est-à-dire par une partie de cornée saine.

Pour procéder à cette ablation, on réalise d'abord une incision généralement circulaire autour de la partie affectée. On retire ensuite la partie incisée de manière connue. Ces opérations sont réalisées sous microscope car elles nécessitent une très grande précision et la découpe ou incision latérale est réalisée au moyen d'un appareil appelé trépan.

On utilise également de tels trépans en chirurgie réfractive, pour pratiquer sur la cornée une incision circulaire, ce qui permet, en contrôlant la suture, d'appliquer à la cornée des contraintes se traduisant par une modification de sa courbure.

L'invention concerne un nouvel appareil du genre trépan.

Il existe actuellement deux types principaux de tels appareils.

Le premier type comprend un couteau à lame cylindrique dont la partie tranchante circulaire est appliquée sur l'oeil et subit une rotation pour inciser.

Par exemple, le brevet français FR-A-1 106 449 décrit un trépan destiné à pratiquer une incision circulaire sur la cornée de l'oeil et comportant à cet effet:

— un support extérieur tubulaire formant enveloppe, présentant un axe et possédant une partie de pied pour son application sur l'oeil,
— un porte-couteau intérieur mobile dans le support, le porte-couteau comportant un corps coaxial au support et portant à sa partie dirigée vers la partie de pied du support un couteau cylindrique également coaxial au support, dont la lame tranchante est constituée par une arête circulaire dirigée vers l'orifice de la partie de pied du support,
— des moyens d'entraînement du porte-couteau par rapport au support, lesdits moyens d'entraînement comportant des moyens d'entraînement du corps à la rotation par rapport au support, autour de l'axe, et des moyens pour lier à ladite rotation une translation du corps par rapport au support parallèlement à l'axe dans le sens de la formation d'une saillie ou dans le sens d'un escamotage de la lame tranchante par rapport à la partie de pied.

Avec les trépans du type décrit par ce document, l'arrêt de la rotation et celui de la translation sont aussi étroitement liés que ces deux mouvements; par conséquent, la translation et la rotation de la lame cylindrique cessent simultanément lorsque la lame forme hors du trépan une saillie de valeur prédéterminée affichée sur l'appareil; en raison de la compressibilité élastique de l'oeil sous l'action de la lame, il est à peu près certain que la profondeur effective de l'incision alors atteinte est inférieure à la valeur de la saillie de la lame, c'est-à-dire inférieure à la profondeur prédéterminée affichée sur l'appareil; il existe en outre des risques importants pour que cette profondeur soit irrégulière, en raison des contraintes de torsion et de cisaillement que subit la cornée lors de son incision par la lame tournant et se déplaçant en translation.

Le brevet américain US-A-2 838 050 décrit un autre exemple de réalisation d'un trépan de ce premier type; ce trépan est étroitement apparenté à celui que décrit FR-A-1 106 449, si ce n'est que la partie de pied est constituée par un tampon interne à la lame cylindrique et prenant appui sur la partie de cornée intérieure à l'incision, et il présente les mêmes inconvénients.

Ces dispositifs connus du premier type sont en outre de maniement très délicat, car ils ne sont pas retenus sur l'oeil et peuvent glisser ou s'incliner. La profondeur de l'incision est de ce fait peu précise et peut être de valeur inégale si, pendant l'opération, l'appareil a pris une inclinaison.

Ils présentent toutefois l'avantage de produire à coup sûr une incision continue en raison de la forme cylindrique de la lame.

Le second type de ces appareils connus est décrit par la demande de brevet français FR-A-2 364 646 et comporte une lame pointue et tranchante latéralement, qui est fixée de manière réglable axialement sur un support possédant un appui sur l'oeil, à l'extérieur de la ligne d'incision, et une lentille transparente interne autour de laquelle la lame peut être animée en rotation. Dans ce cas, l'incision est réalisée progressivement par une alternance de rotations et d'enfoncements de la lame, et il est possible d'uniformiser la profondeur de pénétration de cette dernière à une valeur prédéterminée en faisant tourner la lame sans translation de celle-ci.

Toutefois, cette possibilité est le résultat d'une dissociation totale des moyens provoquant la rotation de la lame et des moyens provoquant sa translation, sans possibilité d'action simultanée sur ces moyens distincts du fait que le chirurgien, tenant le trépan d'une main, ne peut consacrer que son autre main au déplacement de le lame et qu'il est hors de question de faire intervenir un autre opérateur à cet effet, compte tenu d'une part des dimensions du trépan et d'autre part des risques inadmissibles de déplacement de ce dernier et que cela pourrait entraîner; dans cet art antérieur, par conséquent, les rotations et les translations sont nécessairement alternées, avec un risque important de déplacement du trépan par rapport à l'oeil compte tenu de la nécessité, pour le chirurgien, d'agir alternativement sur des moyens différents pour provoquer tantôt une augmentation de la profondeur de coupe, tantôt la rotation du couteau.

Si un mouvement de l'appareil par rapport à l'oeil se produit au cours de l'incision, on peut ne pas obtenir de coïncidence entre le point de départ de la lame et son point d'arrivée après sa rotation. L'incision est alors à terminer manuellement et la forme de la découpe n'est plus régulière.

Le but de l'invention est de réaliser une incision parfaitement circulaire et de profondeur précise et égale sur tout son périmètre, de préférence avec réglage avant la pose de l'appareil sur l'oeil, couteau rétracté.

A cet effet, l'invention propose un trépan destiné à pratiquer au moins une incision circulaire sur la cornée de l'oeil et comportant comme il est connu en soi de FR-A-1 106 449:

— un support extérieur tubulaire formant enveloppe, présentant un axe et possédant une partie de pied pour son application sur l'oeil,

— un porte-couteau intérieur mobile dans le support, le porte-couteau comportant un corps coaxial au support et portant à sa partie dirigée vers la partie de pied du support un couteau cylindrique également coaxial au support, dont la lame tranchante est constituée par une arête circulaire dirigée vers l'orifice de la partie de pied du support,

— des moyens d'entraînement du porte-couteau par rapport au support, lesdits moyens d'entraînement comportant des moyens d'entraînement du corps à la rotation par rapport au support, autour de l'axe, et des moyens pour lier à ladite rotation une translation du corps par rapport au support parallèlement à l'axe dans le sens de la formation d'une saillie ou dans le sens d'un escamotage de la lame tranchante par rapport à la partie de pied, ce trépan étant caractérisé selon l'invention en ce que des moyens sont prévus pour entraver ladite translation en autorisant ladite rotation lorsque le corps parvient dans une position prédéterminée, de préférence préréglable à volonté, par rapport au support, lors de ladite translation dans le sens de la formation d'une saillie.

Ainsi, comme il ressortira de la suite de la description, dans le contexte connu d'un trépan mettant en oeuvre une lame cylindrique soumise à des mouvements conjoints de rotation et de translation, la présente invention offre la possibilité de faire se succéder directement, par action de l'opérateur sur des moyens d'entraînement uniques, une phase de rotation de la lame avec translation puis une phase de rotation sans translation avec transition entre les deux phases lorsque la lame forme une saillie prédéterminée par rapport à d'autres éléments du trépan, ce qui permet d'uniformiser la profondeur de l'incision obtenue.

Notamment, le trepan selon l'invention permet de pratiquer sur la cornée au moins deux incisions concentriques, avec ou sans prélèvement de l'anneau intermédiaire, pour modifier la courbure de la cornée; il permet également, grâce à sa lame circulaire, de prélever un greffon conservé.

Selon un mode de réalisation, le corps est pourvu à sa surface extérieure d'un filetage coopérant avec un taraudage intérieur porté par le support, et lesdits moyens d'entraînement sont en prise directement avec le corps; alors, avantageusement, ledit taraudage intérieur est porté par un élément mobile en rotation autour de l'axe dans le support, entre deux butées écartées angulairement d'un angle prédéterminé et des moyens sont prévus entre le corps et ledit élément d'une part, et entre ledit élément et le support d'autre part, pour que les forces de frottement tendant à s'opposer à la rotation du porte-couteau par rapport audit élément soient différentes des forces de frottement tendant à s'opposer à la rotation dudit élément par rapport au support; ou encore, ledit taraudage intérieur est porté par un élément mobile autour de l'axe dans le support, et des moyens sont prévus entre le corps et ledit élément d'une part et entre ledit élément et le support d'autre part, pour que les forces de frottement tendant à s'opposer à la rotation du porte-couteau par rapport audit élément soient inférieures aux forces de frottement tendant à s'opposer à la rotation dudit élément par rapport au support et que ledit élément soit néanmoins susceptible d'une rotation par rapport au support, lors d'un entraînement du corps à la rotation dans le sens de la formation d'une saillie, mais soit immobilisé part rapport au support lors d'un entraînement du corps à la rotation en sens inverse.

De préférence, le support et le porte-couteau sont chacun équipés d'une première et d'une seconde butée, les premières butées étant portées de manière fixe par le support et le porte-couteau pour constituer lors de leur venue en butée une limite à la rétraction du porte-couteau dans le support, l'une des secondes butées étant portée de manière fixe par l'une des pièces (support ou porte-couteau) susdites tandis que l'autre des secondes butées est portée de manière réglable par l'autre pièce, pour constituer lors de leur contact mutuel une limite réglable au mouvement du porte-couteau par rapport au support en direction de l'oeil, cette limite correspondant à la profondeur de l'incision à réaliser, un dispositif d'affichage de la position de ladite butée par rapport à la pièce, constitué par un index et une graduation exprimée en unités de profondeur d'incision, étant associé auxdites pièce et butée réglable.

A cet égard, un mode préféré de réalisation consiste en ce que la pièce susdite est le porte-couteau, pourvu d'un second filetage et d'une couronne crantée qui lui est solidaire, tandis que la butée réglable est constituée par un épaulement d'une douille filetée , coopérant avec ledit second filetage, pourvue d'un cliquet élastique coopérant avec ladite couronne crantée.

En outre, la graduation du dispositif d'affichage susdit est portée par les saillies de la couronne crantée tandis que l'extrémité du cliquet susdit définit, avec l'extrémité d'un masque solidaire de la douille, une fenêtre de lecture de ladite graduation constituant ledit index.

Selon une autre caractéristique de l'invention, le support est pourvu d'une paroi fixe intérieure au corps du porte-couteau, formant élément de fixation pour une lentille amovible, complétée le cas échéant par une lentille annulaire interposée entre le couteau et le support.

Enfin, la partie de pied du support susdit est démontable et le couteau interchangeable.

L'invention sera mieux comprise au cours de la description donnée ci-après à titre d'exemple purement indicatif et non limitatif, qui permettra d'en dégager d'autres avantages et caractéristiques.

Il sera fait référence aux dessins annexés dans lesquels:

la figure 1 est une vue générale d'un mode de réalisation d'un trépan selon l'invention, en coupe par un plan incluant son axe,

les figures 2 et 3 illustrent un couteau mis en oeuvre dans l'invention, respectivement en coupe par un plan incluant l'axe, tel que le plan II-II de la figure 3, et en vue de dessus suivant l'axe,

les figures 4, 5, 6 illustrent l'écrou de blocage du couteau selon l'invention, respectivement en vue de dessous suivant l'axe, en coupe par un plan incluant l'axe, tel que le plan V-V de la figure 4, et en élévation latérale,

les figures 7 et 8 illustrent la douille de réglage de la profondeur d'incision, respectivement en vue de dessous suivant l'axe et en coupe par les deux demiplans repérés en VIII-VIII à la figure 7,

les figures 9 et 10 illustrent la couronne crantée de verrouillage et d'affichage de la profondeur d'incision coopérant avec la douille des figures 7 et 8, respectivement suivant l'axe et en élévation latérale,

la figure 11 est un schéma d'une variante de réalisation d'un détail de l'invention, en coupe par un plan incluant l'axe,

la figure 12 montre une vue partielle, analogue à celle de la figure 1, d'une variante de réalisation de l'invention,

la figure 13 montre une vue en coupe selon le plan XIII-XIII de la figure 12,

la figure 14 montre schématiquement un détail de la figure 13.

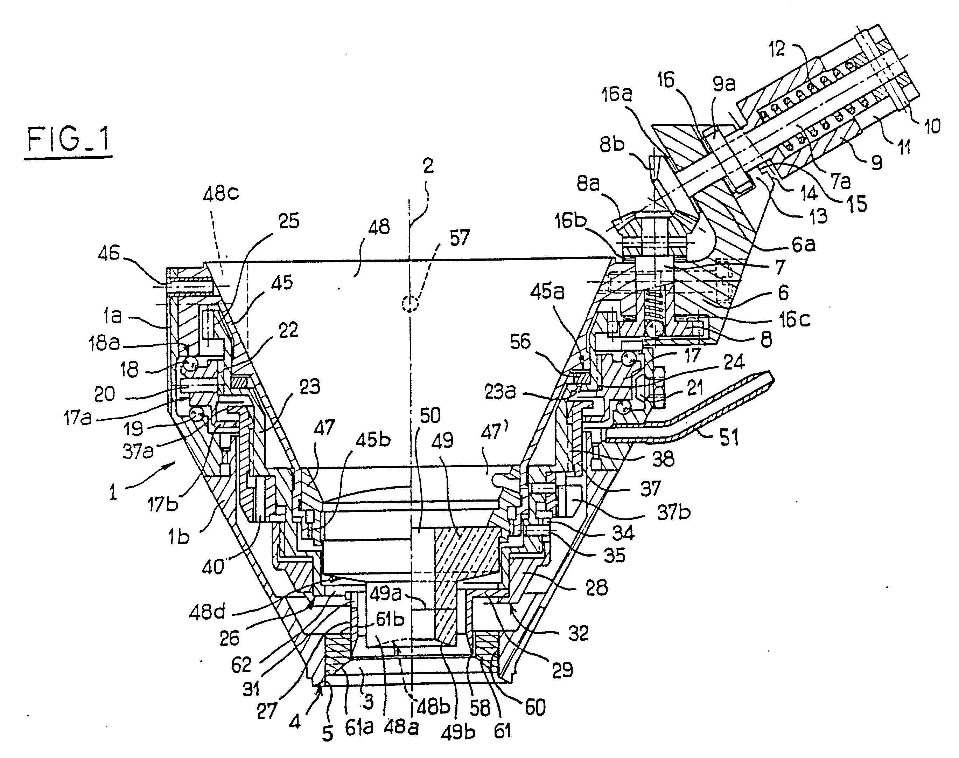

En se reportant à la figure 1, on voit un trépan qui comporte un support extérieur 1 comportant une partie postérieure 1a de forme sensiblement cylindrique et une partie de pied 1b de forme sensiblement conique vissée et centrée sur la partie 1a. Ce support est creux et possède un axe longitudinal 2. La partie 1b est ouverte en 3. L'ouverture 3 est bordée d'une face antérieure 4 formant une couronne sensiblement sphérique pour s'appuyer sur l'oeil du patient et est pourvue d'une pluralité de griffes 5 présentant des dimensions suffisantes pour empêcher le glissement du trépan sur l'oeil, mais néanmois suffisamment faibles pour ne pas marquer l'oeil;

dans une variante, les griffes 5 sont circonférentiellement réparties à un pas compatible avec le nombre des points de suture à réaliser lors de la mise en place du greffon, et présentent des dimensions telles qu'elles forment sur l'oeil des marques qui indiquent les points où est à réaliser la suture.

La partie postérieure 1a du support 1 est équipée latéralement d'un palier 6 dans lequel une tige 7 parallèle à l'axe 2 est susceptible de tourner. A l'une de ses extrémités, la tige 7 porte un pignon 8 tandis que son autre extrémité est équipée d'un pignon conique de renvoi d'angle 8a, en prise avec un pignon analogue 8b solidaire de l'extrémité inférieure d'une tige 7a, de préférence oblique par rapport à l'axe 2, susceptible de tourner dans un deuxième palier latéral 6a de la partie postérieure 1a du support 1; l'autre extrémité de la tige 7a est équipée d'un bouton de manoeuvre manuelle 9 calé en rotation sur la tige 7a au moyen d'une goupille 10. Le bouton 9 est susceptible de coulisser le long de la tige 7a et d'être guidé dans son coulissement par des encoches 11 dans lesquelles les extrémités de la goupille 10 peuvent se déplacer. Un ressort 12 tend à pousser vers le bas le bouton 9, qui comporte à sa partie inférieure une partie 9a présentant des pans latéraux. Lorsque cette partie 9a est dans la position de la figure, elle est placée et maintenue sous l'effet du ressort 12 dans un logement du palier 6a, dans lequel elle peut tourner. Ce logement possède une ouverture supérieure 13 présentant une forme complémentaire de celle de ladite partie 9a pour autoriser cette partie 9a à traverser cette ouverture, si elle est correctement orientée angulairement, lorsque l'on tire sur le bouton 9 à l'encontre de l'effet du ressort 12. Au-delà et de chaque côté de l'ouverture 13, le palier 6a se termine par une face extérieure 14 dans laquelle on a ménagé un creux 15 présentant également une forme complémentaire de celle de la partie 9a mais toutefois orienté perpendiculairement à l'ouverture 13 pour recevoir la partie 9a du bouton dans une position dans laquelle cette partie 9a est bloquée en rotation, et dans laquelle le pignon 8 ne peut donc plus tourner. Ce verrouillage peut être à tout instant libéré en soulevant le bouton 9 à l'encontre de l'effet du ressort 12 et en replaçant la partie 9a dans son logement situé sous l'ouverture 13, dans lequel elle peut librement tourner. On notera que dans ce logement, sous la partie 9a, on a placé une garniture 16 qui assure un frottement minimal du bouton 9 lors de sa rotation; de même, entre le palier 6a et le pignon 8b et entre le palier 6 et les pignons 8a et 8 sont intercalées des garnitures, respectivement 16a, 16b, 16c, assurant un frottement minimal et, dans le cas des garnitures 16b et 16c, une étanchéité de la tige 7 par rapport au palier 6.

Selon une variante non représentée, la tige 7a porte en outre un pignon susceptible de s'engrener avec le pignon de sortie d'un ensemble moto-réducteur par exemple à avance pas-à-pas, pour permettre la motorisation du trépan; dans ce cas, le moteur peut avantageusement être commandé et/ou contrôlé par un micro-processeur, qui affiche à chaque instant une valeur de descente de l'arête tranchante du couteau, ou la profondeur d'incision atteinte.

A l'intérieur de la partie 1a postérieure du support, on a placé une bague annulaire 17 qui peut y tourner. Cette bague est immobilisée axialement dans le support par deux paliers à billes 18, 19 qui peuvent être remplacés ou complétés par des surfaces à faible coefficient de frottement; en particulier, entre les billes respectives des paliers 18 et 19 peuvent être intercalés des tronçons d'un jonc torique en un matériau tel que le P. T. F. E. joignant deux à deux les billes voisines pour empêcher la pénétration, entre elles, de poussières susceptibles d'entraver leur roulement. La bague 17 possède une surface extérieure 17a de laquelle fait radialement saillie un pion radial 20 de faible largeur. Par ailleurs la partie 1a du support possède un pion 21 faisant saillie intérieurement en regard de la surface extérieure 17a pour servir de butée au pion 20. Ainsi, la rotation de la bague 17 dans le support est limitée à une valeur un peu inférieure à 360°. La bague 17 porte en outre un taraudage intérieur 22.

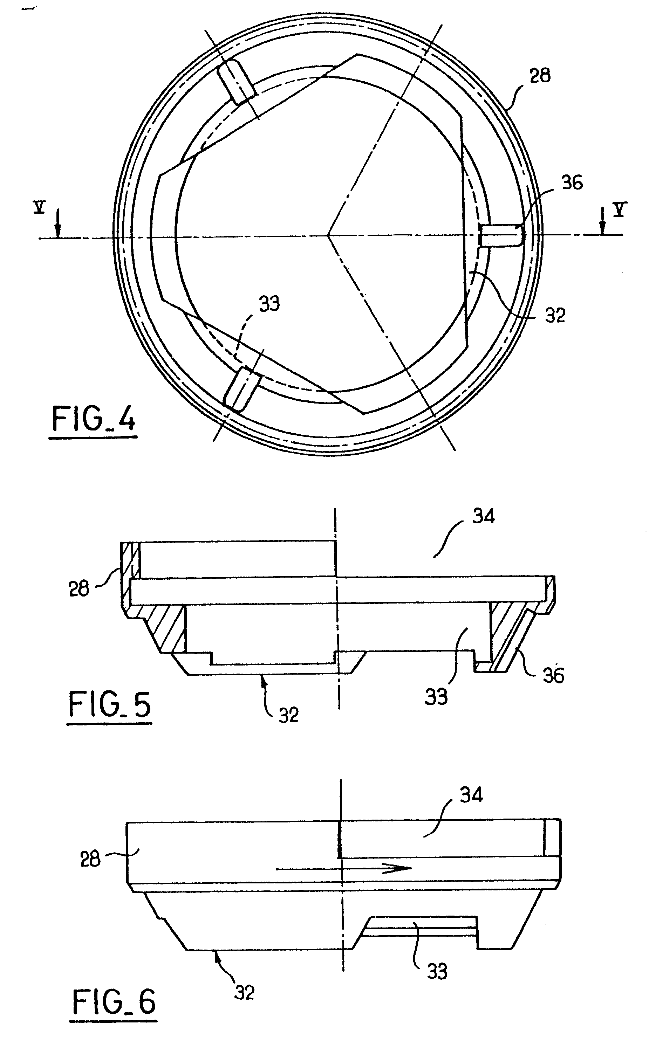

Le taraudage 22 est destiné à recevoir un filetage extérieur 24 réalisé sur le corps 23 d'un porte-couteau. La forme générale de ce porte-couteau est conique pour correspondre à la forme générale du support. A sa partie postérieure (ou supérieure), le corps 23 est pourvu d'une couronne dentée 25 qui engrène avec le pignon 8 susdit. La partie inférieure (ou antérieure) du corps 23 comporte une face transversale 26 qui forme face d'appui pour un couteau 27 pour l'essentiel cylindrique de révolution autour de l'axe 2, maintenu sur le porte-couteau au moyen d'un écrou de serrage et de centrage 28, présentant un filetage par lequel il est vissé sur un filetage du porte-couteau; avantageusement, une flèche gravée à l'extérieur de l'écrou 6 indique le sens de rotation au vissage (figure 6).

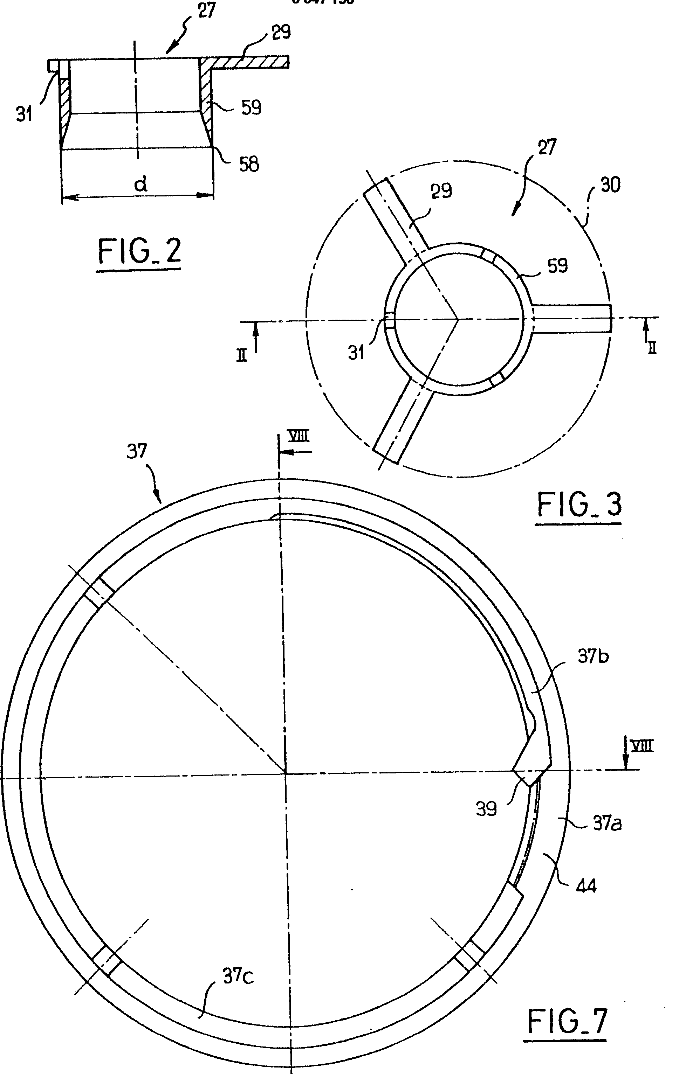

Les figures 2 et 3 illustrent un mode de réalisation du couteau selon l'invention, tandis que les figures 4, 5, 6 montrent en détail l'écrou de blocage de ce couteau sur la face d'appui 26 susdite. On voit sur ces figures que le couteau 27 possède une paroi 59 pour l'essentiel cylindrique de révolution se terminant par une arête tranchante circulaire 58 définissant la lame du couteau, et un épaulement postérieur 29 défini par une pluralité de branches radiales régulièrement réparties, ici au nombre de trois, dont les extrémités sont arrondies et situées sur un même cercle virtuel 30. On notera également la présence d'orifices ou d'encoches 31 traversant radialement la paroi cylindrique 59 du couteau. On peut fixer indifféremment sur le trépan de l'invention des couteaux de dimensions différentes, selon notamment les caractéristiques géométriques de l'oeil à traiter. Le diamètre d de l'arête tranchante 58 pourra, par exemple, varier entre 7 et 9,5 mm avec un pas de 0,25 mm, la découpe de l'épaulement 20 étant identique d'un couteau à l'autre.

En munissant les couteaux de filetages complémentaires ou de moyens d'emboîtement mutuel appropriés, on pourra également disposer deux couteaux concentriquement, l'un dans l'autre, dans des positions relatives parfaitement prédéterminées notamment quant à leurs arêtes tranchantes, pour pratiquer simultanément sur l'oeil deux incisions circulaires concentriques en chirurgie réfractive.

L'écrou 28 comporte quant à lui une face inférieure 32 possédant une découpe sensiblement ou approximativement complémentaire de celle de l'épaulement 29 du couteau de façon à permettre d'introduire cet épaulement dans un logement intérieur cylindrique 33 de l'écrou 28. Ce logement 33 présente un diamètre égal au diamètre du cercle 30, ce qui permet, en vissant l'écrou, de décaler sa découpe par rapport à celle de l'épaulement 29 du couteau et de serrer cet épaulement contre la face 26 (qui comportera de préférence un léger lamage de forme complémentaire de la forme de l'épaulement 29 pour retenir le couteau à l'encontre d'une rotation lors du serrage de l'écrou). La face périphérique cylindrique de ce logement 33 sert en même temps de centrage de l'écrou sur le corps 23, c'est-à-dire par rapport à l'axe 2.

Par ailleurs, l'écrou 28 possède au niveau de son filetage une partie évidée ou décrochée 34 dont les bords espacés angulairement d'environ 100 à 120° coopèrent avec un pion radial 35 (figure 1) porté par le corps 23. La mise en place de ce pion est bien entendu réalisée après réglage de l'écrou de manière que la rotation partielle qu'il permet autorise le serrage et le desserrage du couteau et son extraction au travers de la découpe de l'écrou. Cette disposition rend indémontable l'écrou 28 et facilite la mise en place du couteau. Des encoches 36 externes à l'écrou permettent sa manoeuvre au moyen d'une clef conçue à cet effet.

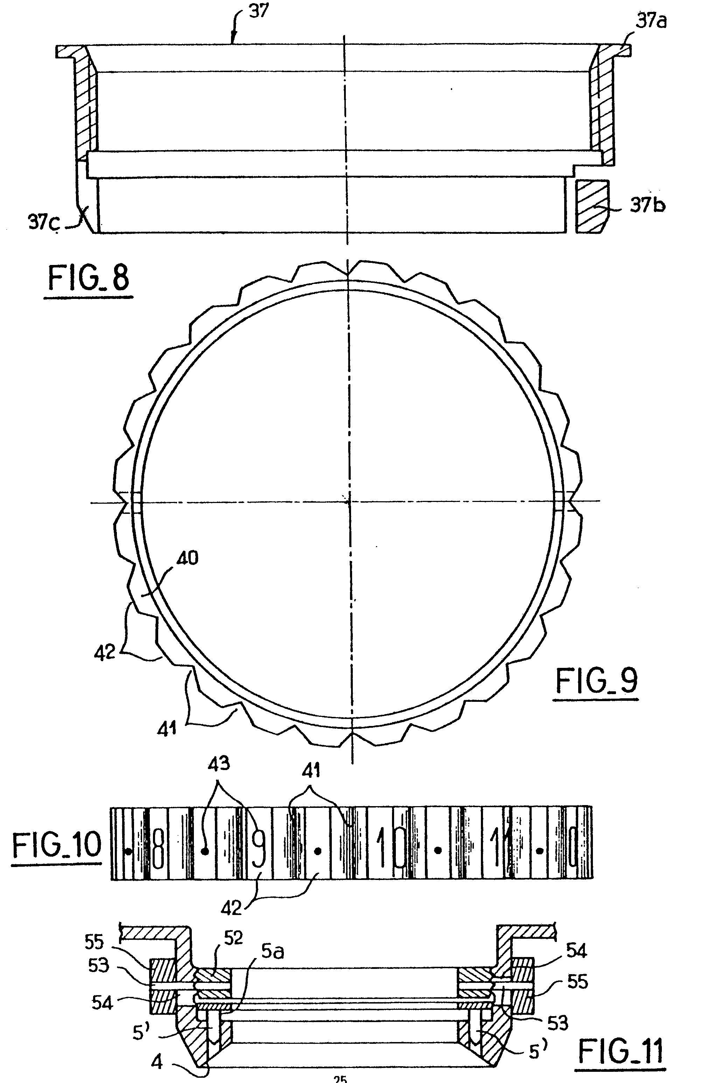

Revenant à la figure 1, on voit qu'entre la couronne 25 et la face 26 le corps 23 du porte-couteau, ce dernier est équipé d'une douille 37 munie d'un filetage intérieur par lequel elle est vissée sur un filetage 38 du corps 23. Cette douille, qui est représentée en vue de dessous sur la figure 7 et en coupe sur la figure 8, comporte une collerette supérieure 37a et un cliquet inférieur 37b qui est un bras élastique curviligne taillé dans une jupe 37c prolongeant la douille dans sa partie inférieure. Ce bras possède une protubérance radiale 39 interne qui coopère avec les encoches d'une couronne crantée 40 solidaire du porte-couteau (en une seule pièce ou rapportée). Cette couronne est représentée en détail aux figures 9 et 10. Entre ses encoches 41, elle posède des saillies 42 qui portent des repères 43 indiquant par exemple en dixièmes et demi-dixièmes de millimètre la profondeur d'incision.

La jupe 37c de la douille 37 définit avec le cliquet 37a une fenêtre 44 (figure 7) dans laquelle n'apparaît qu'un seul des repères 43, correspondant à la profondeur d'incision désirée.

On voit en outre sur la figure 1 que le support 1 possède une paroi intérieure 45 attelée à la partie 1a au moyen d'éléments de fixation 46 (pions). Cette paroi intérieure d'une part constitue le chemin de roulement supérieure 18a des billes 18, d'autre part comporte un épaulement 45a réalisé en regard d'un épaulement 23a du porte-couteau pour constituer des butées fixes de limitation de la rétraction du porte-couteau dans le support; enfin, elle est filetée intérieurement à son extrémité antérieure 45b pour recevoir une pièce 47 porte-lentille.

Avantageusement, entre les épaulements 45a et 23a est intercalée une bague 56 d'un matériau à faible coefficient de frottement, telle qu'une bague fendue de P. T. F. E., pour éviter un coincement du corps 23, en position extrême de rétractation, contre la paroi 45 par frottement mutuel des épaulements 45a et 23a.

La position extrême de rétractation du corps 23 par rapport à la paroi 45 et à la partie 1a du trépan peut également être définie, en complément ou en remplacement de la venue des épaulements 45a et 23a en butée, le cas échéant par l'intermédiaire de la bague 56, par venue en contact de l'une des dents de la couronne 25 du corps 23, prolongée au-dessus de cette couronne (par rapport aux autres dents), avec un pion 57 porté par la paroi 45 et formant saillie radialement vers l'extérieur par rapport à celle-ci, sur le passage obligé de cette dent lorsque le corps 23 parvient en position de rétractation extrême; la distance, mesurée parallèlement à l'axe 2, dont la dent est ainsi prolongée par rapport aux autres dents de la couronne 25, est inférieure au pas du taraudage 22 et du filetage 24 de telle sorte que, après que le corps 23 ait effectué un tour par rapport à la bague 17 à partir de la position de rétractation extrême ainsi définie, la dent prolongée échappe au pion 57 et n'entrave pas le mouvement de descente du corps 23.

Sur la partie gauche de la figure 1, la pièce porte-lentille 47 porte une lentille 48 conique remplissant pratiquement complètement l'espace intérieur défini par la paroi 45. Cette lentille 48, attelée à demeure à la pièce 47 par collage ou analogue, possède une extension antérieure 48a de diamètre inférieur co-axiale au couteau, à l'intérieur de celui-ci, et terminée par une face concave 48b. Lorsque la pièce 47 est vissée à fond dans l'extrémité 45b susdite, la face 48b est placée approximativement ou sensiblement dans le prolongement sphérique, à l'intérieur du couteau, de la face 4 de la partie de pied du support, en même temps que celle-ci pour épouser la surface de l'oeil. Des encoches 48c prévues dans la lentille permettent son montage ou démontage au moyen d'une clef. Dans une variante non représentée, on aura prévu que la pièce 47 enveloppe la lentille 48 jusqu'à sa partie supérieure, les encoches susdites étant alors réalisées dans l'extrémité supérieure de ladite pièce 47 ainsi prolongée.

Sur la partie de droite de la figure 1, on a représenté en coupe un autre type de lentille 49 solidaire d'une pièce 47' analogue à la pièce 47. Cette lentille 49 possède un évidement central 50 qui, entouré par une face antérieure 49b de courbure et de position identiques à celles de la face 48b susdite, permet de pratiquer des opérations sur des cornées non sphériques, présentant par exemple une déformation en cône. Cette lentille possède deux fils 49a qui se croisent au centre de l'espace 50 pour former réticule de centrage.

On voit donc que, dans le trépan selon l'invention, la lentille 48—49 est fixe par rapport au support 1.

Avantageusement, comme il est illustré, l'extension antérieure 48a de la lentille 48 (ou l'extension analogue de la lentille 49) est reliée à la partie supérieure de la lentille par une face 48b orientée approximativement transversalement par rapport à l'axe 2, et tournée vers le bas, c'est-à-dire vers le couteau 27, au-dessus de celui-ci; dans l'exemple illustré, cette face 48d présente une forme tronconique de révolution autour de l'axe 2, le sommet du cône étant tourné vers le bas, pour faciliter l'évacuation de l'air lorsque, lors de l'utilisation d'une lentille du type illustré en 47 dans la partie gauche de la figure 1, c'est-à-dire d'une lentille ne présentant pas d'évidement central tel que 50 et délimitant par conséquent un volume fermé avec le support 1 et la paroi intérieure 45 du trépan d'une part, l'oeil du patient d'autre part, on met l'intérieur du trépan en dépression par rapport à l'extérieur grâce à des dispositions qui seront décrites plus loin; la face 48b pourrait toutefois présenter d'autres formes, et par exemple une forme plane ou une forme concave analogue à celle de la face 48b.

Complémentairement, lorsque le diamètre extérieur de la paroi cylindrique 59 du couteau 27 est suffisamment inférieur au diamètre intérieur de la surface annulaire 4, qui définit le diamètre intérieur minimal de la partie 1b, il est avantageux de prévoir, entre la paroi cylindrique 59 et la face cylindrique intérieure 60 de la partie 1b adjacente à la surface 4 de celle-ci, une lentille annulaire 61 fixe par rapport à la partie 1b et ainsi placée en regard de la face 48d de la lentille 48 (ou de son homologue de la lentille 49), par l'intermédiaire d'espaces 62 subsistant entre la paroi cylindrique 59 du couteau, les bras radiaux définissant l'épaulement 29 de celui-ci, et la périphérie de la découpe de la face 32 de l'écrou 28; l'opérateur dispose ainsi d'une visibilité totale de la cornée du patient, y compris à l'extérieur du couteau circulaire.

Vers l'extérieur, c'est-à-dire vers la cornée, la lentille annulaire 61 présente avantageusement une face concave 61a prolongeant la face 48b de la lentille 48 jusqu'à la face 4 pour s'appuyer sur l'oeil simultanément à celles-ci, en l'épousant; vers l'intérieur du trépan, elle peut présenter une face 61b plane, perpendiculaire à l'axe 2 comme il est illustré, ce qui permet à l'ensemble formé par la lentille 48 (ou 49) et la lentille 61 de fournier deux grossissements différents respectivement pour l'intérieur et l'extérieur du couteau circulaire, ou une face présentant une forme complémentaire de celle de la face 48d pour fournir un grossissement uniforme.

Enfin on notera que le support 1 est pourvu d'embouts 51 (deux, dont un seulement est visible sur la figure) pour relier l'espace intérieur du trépan (entre parois 45 et parois 1a, 1b) à, d'une part, une source de fluide, d'autre part, une source d'aspiration permettant de créer un vide partiel dans le champ opératoire, pour plaquer ainsi le trépan sur l'oeil et l'immobiliser au mieux, et une circulation de fluide aseptique dans ce champ, lorsqu'on utilise une lentille non évidée du type illustré par exemple en 48 dans la partie gauche de la figure 1; cette mise en dépression et cette circulation sont facilitées par une conformation appropriée de l'espace intérieur du trépan.

Avantageusement, dans ce cas, un microprocesseur contrôle et pilote en continu la pression régnant à l'intérieur du trépan pour détecter la présence d'éventuelles bulles d'air et contrôler leur élimination.

Avantageusement, dans le cas du mode de réalisation préféré illustré, les indices de réfraction respectifs des lentilles 48 et 61 sont différents, inférieur pour la lentille principale 48 et supérieur pour la lentille 61; à titre d'exemple non limitatif, la lentille 48 peut présenter un indice de réfraction de l'ordre de 140 à 150, et la lentille 61 un indice de réfraction de l'ordre de 180.

Dans une variante de réalisation d'un détail représenté schématiquement à la figure 11, des griffes 5' du type évoqué plus haut, destinées à former sur l'oeil des marques indiquant l'emplacement des points de suture, sont supportées par une rondele 5a qui peut coulisser librement dans le nez du support 1b. Une rondelle 52 est vissée dans le nez du support avec un pas important (2,5 à 3 mm) et est reliée par des pions 53 au travers d'ouvertures 54 ménagées dans la partie 1b à une bague de manoeuvre externe 55. Lorsqu'on remonte la bague 55 (en dévissant la rondelle 52), on libère un espace au-dessus de la rondelle 5a et, au contact de l'oeil, les griffes 5' peuvent remonter et s'escamoter dans la surface 4. En faisant tourner d'un quart de tour, par exemple, la bague 55, on fait sortir les griffes de 6 à 7 dixièmes de millimètre et on les maintient enfoncées dans l'oeil sous l'action de la rondelle 52.

On expliquera maintenant le fonctionnement du trépan selon l'invention. On supposera que les réglages initiaux et définitifs ont été réalisés au moment du montage de l'appareil. Ces réglages doivent conduire aux résultats suivants:

— quand la face 45a et la face 23a susdites sont en contact par l'intermédiaire de la bague 56, et/ou la dent prolongée de la couronne 25 en contact avec la butée 57, l'arête tranchante du couteau se trouve à l'intérieur du trépan en-deçà de la surface 4, 48b ou 49b, 61a;

— quand l'affichage réalisé par le moyen de la douille filetée 37 et la couronne crantée 40 est ou correspond au zéro, le porte-couteau 23 ne peut descendre que jusqu'au moment où l'arête tranchante du couteau est contenue dans ladite surface 4, 61a, 48b ou 49b.

L'opérateur a disposé sur la face 26 du porte-couteau 23 le couteau 27 et l'y a bloqué au moyen de l'écrou 28. Cette opération n'a été possible qu'après avoir retiré la partie 1b du support de la partie 1a. Pour réaliser ce blocage, il aura utilisé une clef agissant sur les encoches 36 de l'écrou 28 en ayant au préalable placé le bouton 9 dans sa position de verrouillage ou blocage total de l'appareil.

Il agit ensuite sur la douille 37 en la vissant (ou dévissant) sur le corps 23 jusqu'à obtenir dans la fenêtre la profondeur d'incision désirée. Cette rotation est possible car le cliquet 37b, souple, saute d'encoche en enchoche sur la couronne crantée 40 en glissant sur leurs pentes (quel que soit le sens de rotation). Ces réglages effectués, la partie 1b est revissée sur la partie 1a. On remarquera (figure 1) que le dos du cliquet 37b est alors très voisin d'une surface intérieure cylindrique de la partie 1b, qui interdit au cliquet de sortir complètement de l'encoche où il a été placé sous un effort de rotation relative du corps 23 et de la douille 37. La douille 37 est ainsi rendue parfaitement solidaire en rotation du porte-couteau.

On aura noté qu'en réalisant cette opération de réglage on fixe en fait la hauteur axiale de l'épaulement 37a de la douille 37 par rapport au corps 23. Or, il faut remarquer que cet épaulement 37a est susceptible de prendre appui sur une extension annulaire radiale interne 17b de la bague 17 montée à rotation dans le support. Lorsque cet appui est réalisé, la limite de la descente du corps 23 par rapport au support 1 est atteinte. On voit donc que plus on descend la collerette (ou épaulement) 37a par rapport au corps 23 et plus vite on atteindra cette limite et donc plus courte sera la sortie du couteau par rapport au support. La position de la douille 37 sur le corps 23 est donc bien en relation avec la profondeur d'incision à obtenir.

L'opérateur ensuite déverrouille le bouton 9 et actionne manuellement le pignon 8 pour »remonter« le corps 23. La rotation du pignon 8 entraîne la rotation de la couronne 25 et donc celle du corps 23. Les forces de frottement existant naturellement au niveau des filetages 22, 24 ont tendace à transmettre cette rotation à la bague 17 montée sur paliers axiaux dont la résistance par frottement est nettement inférieure à celle des filetages. Si cependant tel n'est pas naturelement le cas, on aura prévu pour respecter cette hiérarchie un frein entre le corps 23 et la bague 17 du type frein d'écrou non représenté, réalisé par exemple sous la forme d'une lame élastique s'appuyant par courbure sur les filets 24 du corps 23. Si les butées 20 et 21 coopèrent pour empêcher la rotation dela bague 17, le corps 23 se dévisse et monte entraînant le couteau 27 dans le support, jusqu'à ce que les faces 45a, et 23a soient en contact. On peut alors reverrouiller le bouton 9.

Le chirurgien place ensuite le trépan sur l'oeil à opérer et procède à son centrage à l'aide d'un repère prévu à cet effet sur la lentille 48 ou du réticule à fils de la lentille 49. Dans la version à griffes 5' rétractables, il procédera à cette mise en place après avoir rentré les griffes. Grâce à ces moyens, la précision de mise en place peut être très grande, la lentille étant un élément fixe du trépan et reposant directement sur l'oeil. Le trépan est alors agrippé par ses griffes 5 ou 5' à la surface de l'oeil. L'assujettissement est affermi par la mise en marche du dispositif d'aspiration-irrigation qui extrait l'air de l'espace fermé par le porte-couteau, le support 1 et l'oeil et le remplace par un sérum physiologique.

On procède ensuite à la manoeuvre du bouton 9 pour faire descendre le porte-couteau. Il faut noter à cet égard que la rotation du pignon 8 entraîne tout d'abord la rotation du corps 23 et de la bague 17 ensemble par les frottements des filetages sur un peu moins de 360° pour la roue dentée 25. Les butées 20 et 21 sont alors à nouveau en contact par leurs autres faces et la poursuite de la rotation du bouton 9 entraîne le vissage dans la bague 17 du corps 23. Le couteau 27 tourne et descend, atteint l'oeil et incise la cornée. Pendant cette opération, le sérum en circulation passe entre la lentille et la lame tranchante par les orifices 31 du couteau et entraîne tout ce qui pourrait nuire à la surveillance de l'opération (sang, débris, etc.) au travers de la lentille et du microscope qui est placé derrière. La descente rotative du couteau est stoppée par le contact de la collerette 37a et de l'extension radiale interne 17b de la bague 17. La profondeur de l'incision choisie est alors atteinte.

Le chirurgien procède alors à une rotation inverse du bouton 9, qui se traduit par une rotation sans remontée du couteau 27 puisque, à ce moment, la bague 17 tourne en même temps que le corps 23. Cette rotation est également un peu inférieure à 360° et s'arrête quand les butées 20 et 21 coopèrent à nouveau. Elle a permis d'égaliser la profondeur de l'incision sur toute sa périphérie en relâchant les contraintes de torsion et de cisaillement qu'a subies la cornée lors de la coupe. Le couteau est alors remonté par la poursuite de la rotation dans le même sens du bouton 9 jusqu'à la butée de fin de course 45a, 23a, 56 et/ou 25, 57.

Dans une variante non représentée, on a prévu, à l'inverse de celle décrite, de rendre le contact des filets 22, 24 le plus doux possible et de placer entre la bague 17 et la partie 1a du support 1 un frein, de manière que les forces de frottement de la bague 17 sur la partie 1b soient supérieures à celles du corps 23 sur la bague 17. Ainsi, lors de l'opération de descente du couteau, la manoeuvre du bouton 9 permettre d'abord de descendre le couteau jusqu'à la profondeur requise puis, par une rotation du bouton dans le même sens, d'entraîner la rotation de la bague 17, sans descente du couteau. On voit que, dans cette variante, la réalisation et la finition de l'incision sont réalisées par rotation du bouton 9 dans le même sens. Cette disposition peut présenter un avantage dans le fait qu'elle apporte une petite simplification de manipulation qui évite la possibilité de retirer le trépan avant d'avoir complètement terminé l'opération comme cela peut se produire dans la réalisation décrite ci-avant.

En conclusion, il convient de prévoir des niveaux de frottement assez différents entre les pièces 23 et 17 et 17 et 1b de manière à obtenir une séquence dans la réalisation de la descente rotative et de la rotation sans descente. Le choix de la localisation du plus fort frottement dépendra de l'ordre de la séquence que l'on veut obtenir.

De façon plus générale, tout moyen approprié peut être utilisé pour que les forces de frottement tendant à s'opposer à la rotation de la pièce 23 par rapport à la pièce 17 soient différentes des forces de frottement tendant à s'opposer à la rotation de la pièce 17 par rapport au support 1, afin d'obtenir une telle séquence.

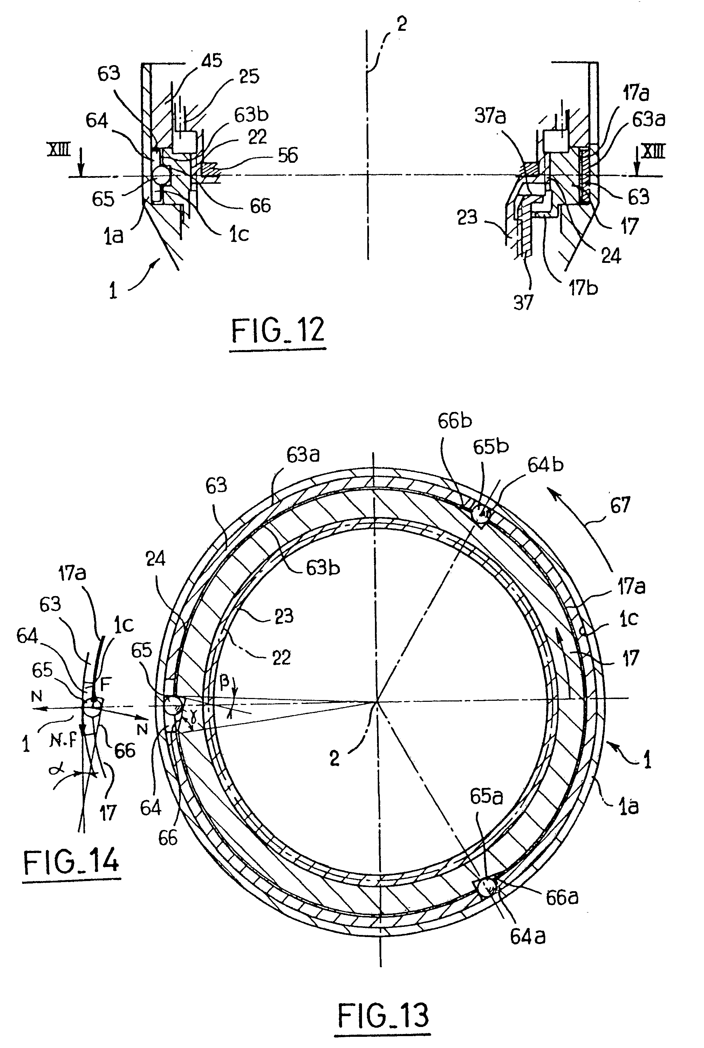

Ainsi, les figures 12 à 14, où l'on a repris les mêmes références qu'aux figures précédentes pour désigner des pièces ou parties analogues, montrent une variante de réalisation dans laquelle les butées 20, 21 et 57 sont supprimées et les paliers à billes 18 et 19 remplacés par des surfaces à faible coefficient de frottement, tout autre mode d'immobilisation axiale de la bague annulaire 17 dans le support 1 avec possibilité de rotation relative autour de l'axe 2 pouvant être choisi dans le cadre de cette variante comme dans celui des précédentes.

Dans cette variante, la face périphérique 17a que la bague 17 présente dans le sens d'un éloignement par rapport à l'axe 2 est cylindrique de révolution autour de celui-ci, et la partie 1a du support 1 présente en regard une face 1c tournée vers l'axe 2, et également cylindrique de révolution autour de celui-ci, avec un diamètre supérieur à celui de la face 17a.

Entre les faces 1c et 17a est intercalée une bague intermédiaire 63 présentant une face périphérique extérieure 63a cylindrique de révolution autour de l'axe 2, avec un diamètre sensiblement identique à celui de la face 1c du support 1, avec laquelle elle est en contact avec possibilité de rotation relative, les forces de frottement tendant à s'opposer à cette rotation étant supérieures aux forces de frottement tendant à s'opposer à la rotation du corps 23 du porte-couteau par rapport à la bague 17, au niveau du filetage 24 et du taraudage 22; par exemple, la bague intermédiaire 63 est fendue en 64, et introduite à force à l'intérieur de la partie 1a du support, pour entrer en contact intime, sous précontrainte, par sa face 63a avec la face 1c de cette partie 1a.

En regard de la face 17a de la bague 17, la bague intermédiaire 63 présente une face périphérique intérieure 63b cylindrique de révolution autour de l'axe 2 avec un diamètre de préférence sensiblement supérieur à celui de la face 17a pour autoriser une rotation relative libre.

La bague intermédiaire 63 est par ailleurs montée libre entre les surfaces à faible coefficient de frottement remplaçant, dans cette variante, les portées de roulement respectives, sur le support 1, des billes 19 et 20 de la variante illustrée à la figure 1.

De façon régulièrement répartie angulairement, la bague 63 est percée radialement de part en part de logements pour des billes dont le diamètre est supérieur à l'épaisseur de la bague 63.

L'un de ces perçages est avantageusement constitué par la fente 64; dans l'exemple illustré, il est en outre prévu deux perçages 64a et 64b, les trois perçages 64, 64a, 64b étant disposés à 120° d'écart angulaire relatif, et recevant des billes à raison d'une par perçage, respectivement 65, 65a, 65b, montées libres.

A l'intérieur de la bague 17, chacune de ces billes est reçue dans une encoche, respectivement 66, 66a, 66b, aménagée dans la face extérieure cylindrique 17a.

Ces encoches sont identiques et présentent, vues en coupe par un plan transversal par rapport à l'axe 2, comme notamment aux figures 13 et 14, une forme approximativement triangulaire définie par deux faces planes parallèles à l'axe 2, par exemple sensiblement ou approximativement perpendiculaires entre elles, dont l'une, située en amont si l'on se réfère à un sens prédéterminé 67 de rotation du corps 23 du porte-couteau par rapport au support 1 dans le sens de la descente du couteau, vers l'oeil du patient, est intégralement située en aval d'un plan joignant l'axe 2 à sa jonction avec la face 17a de la bague 17 en formant avec ce plan un dièdre d'angle $\beta$ inférieur à 90° mais supérieur à 0° et dont l'autre, située en aval de la première est intégralement située en amont d'un plan joignant l'axe 2 sa jonction avec la face 17a de la bague 17, en formant avec ce plan un dièdre d'angle $\gamma$ supérieur à 90° mais inférieur à 180°.

Les dimensions des encoches sont telles que, lorsqu'elle est engagée au maximum dans une telle encoche, la bille correspondante forme saillie par rapport à la face 17a et présente, à l'intérieur du perçage correspondant de la bague intermédiaire 63, une zone située à proximité immédiate de la face 1c de la partie 1a du support sans toutefois entrer en contact avec cette face 1a.

Lorsque l'on entraîne le corps 23 du porte-couteau à la rotation dans le sens 67, notamment à partir d'une position initiale de celui-ci dans laquelle il est en position extrtême de rétractation du couteau, définie par la coopération des faces 23a et 45a, les billes tendent précisément à occuper cette position du fait que les frottements existant au niveau du taraudage 22 et du filetage 24 tendent à provoquer la rotation de la bague 17 conjointement avec le corps 67 alors que des efforts de frottement supérieurs intervenant au niveau des faces en contact 63a et 1c tendent à s'opposer à cette rotation de la bague 17, ce qui amène chaque bille en butée d'une part vers l'aval contre la face aval du perçage correspondant de la bague 63 (sauf éventuellement en ce qui concerne le perçage défini par la fente 64 si l'on se réfère à l'exemple illustré où celle-ci présente dans le sens périphérique des dimensions supérieures à celles des perçages 64a et 64b) et d'autre part vers l'amont contre la face amont de l'encoche correspondante de la bague 17.

Dès que cet état est atteint, le mouvement de descente du corps 23 et du couteau commence, par rotation du corps 23 dans la bague 17 restant quant à elle immobile par rapport au support 1 du fait que les forces de frottement s'opposant à sa rotation sont supérieures à celles que s'opposent à la rotation du corps 23 à l'intérieur d'elle.

Lorsque la profondeur d'incision affichée est atteinte, l'épaulement 37a de la douille 37 vient en butée contre l'épaulement 17b de la bague 17 et la poursuite de l'entraînement du corps 23 à la rotation dans le sens 67 provoque de ce fait une rotation conjointe de la bague 17, et de la bague 63 par l'intermédiaire des billes 65a, 65b en butée, avec friction de la face 63a de celle-ci contre la face 1c de la partie 1a du support; dans le cas de cette variante, par conséquent, la rotation du couteau sans descente s'effectue par entraînement du corps 23 du porte-couteau dans le même sens que lors de la descente, moyennant un effort légèrement supérieur puisque la rotation de la bague 63 par rapport au support 1 se substitue alors à la rotation du corps 23 par rapport à la bague 17.

On notera que l'angle de rotation sans descente du couteau n'est pas limité dans ce cas.

Lorsque l'opérateur désire rétracter le couteau, il inverse le sens de rotation du corps 23 qui, du fait des frottements existant entre le filetage 24 et le taraudage 22 d'une part, les épaulements 37a et 17b d'autre part, tend à entraîner également la bague 17 en sens inverse du sens 67; chaque bille 65, 65a, 65b est alors repoussée vers l'extérieur par un effet de came qui lui est appliqué par la face de l'encoche correspondante placée en aval si l'on se réfère au sens 67, et vient de ce fait au contact sous pression de la face 1c de la partie 1a du support 1.

La figure 14 schématise la décomposition des efforts intervenant alors au niveau de la bille 65, un phénomène identique se produisant au niveau des billes 65a et 65b.

$\alpha$ désigne l'angle que forme ladite face aval de l'encoche 66 (si l'on se réfère au sens 67) avec la tangente à la face 1c au point de contact de la bille avec celle-ci.

F désigne l'effort appliqué à la bille 65 par la bague 17, via l'encoche 66, suivant la bissectrice de l'angle $\alpha$.

Cet effort se traduit par des efforts identiques N appliqués respectivement perpendiculairement à la face 1a (c'est-à-dire radialement) au pont de contact de la bille 65 avec celle-ci et perpendiculairement à ladite face aval de l'encoche 66 (si l'on se réfère au sens 67). On démontre que:

$$N \simeq \frac{F}{\sin \alpha}$$

Le premier de ces efforts N se traduit par un effort de frottement N.f de la bille 65 contre la face 1c, où f désigne un coefficient de frottement.

Par le choix d'un angle $\alpha$ approprié, par exemple de l'ordre de 10° dans l'exemple illustré, on obtient que lors du début de l'entraînement du corps 23 à la rotation en sens inverse du sens 67, il apparaisse ainsi entre les billes 65, 65a, 65b et la surface 1c des efforts de frottement suffisants pour immobiliser la bague 17 par rapport au support 1, en dépit des efforts qui peuvent lui être appliqués par frottement par le corps 23 via notamment les épaulements 37a et 17b, ce qui permet de traduire la rotation du corps 23 immédiatement par la remontée du couteau dans le support 1 vers la position rétractée extrême.

Le trépan selon la présente invention permet de réunir les conditions optimales pour assurer une bonne trépanation de la cornée. En effet, le réglage de la profondeur d'incision est réalisé avant la pose de l'appareil, ce qui élimine un grand nombre de fausses manoeuvres et de déréglages en cours d'opération. La profondeur de l'incision peut être par exemple réglée de 0 à 1,2 mm de cinq en cinq centièmes. De ce fait, on peut pratiquer des trépanations lamellaires, même profondes, car la profondeur de l'incision est uniforme grâce à la rotation sans mouvement axial en fin d'opération.

Il permet d'assurer facilement un bon centrage et une bonne fixation du trépan sur l'oeil en empêchant par ses griffes et son système d'aspiration-irrigation tout glissement, cause de la création d'un double sillon. En outre, ledit système garantit un bon placage de l'oeil sur la face antérieure du trépan et donc une incision toujours verticale. Il permet également d'évacuer le sang si la cornée est vascularisée, évitant les troubles dans le champ de vision de l'opérateur, et d'éviter la plaie de l'iris et du cristallin dans le cas d'une incision perforante. On rapellera également que les empreintes laissées par les griffes marqueront le surjet à réaliser et pourront constituer des avant-trous bien perpendiculaires à la surface de l'oeil sur la majeure partie de l'épaisseur traversée par le fil, ce qui diminue la tendance du fil de surjet à inciser localement les bords de ses orifices de pénétration avec pour conséquence un relâchement de sa tension.

On notera encore que les couteaux, outre qu'ils sont jetables, sont interchangeables. On peut ainsi à l'aide d'un seul trépan pratiquer des trépanations de diamètres différents (par exemple 7 à 9,5 mm de 25 centièmes en 25 centièmes).

Enfin, les lentilles fixées au trépan peuvent être adaptées à la forme de l'oeil malade (par exemple présentant un kératocône) et le trépan dans sa totalité est stérilisable dans un autoclave.

L'invention trouve une application intéressante dans le domaine de la chirurgie opthalmologique.

Elle n'est pas limitée à la description qui vient d'en être donnée mais couvre au contraire toutes les variantes qui pourraient lui être apportées sans sortir de son cadre.

**Revendications**

1. Trépan destiné à pratiquer au moins une incision circulaire sur la cornée de l'oeil, comportant:

— un support extérieur tubulaire (1) formant enveloppe, présentant un axe (2) et possédant une partie de pied (1b) pour son application sur l'oeil,
— un porte-couteau intérieur mobile dans le support, le porte-couteau (23, 28) comportant un corps (23) coaxial au support (1) et portant à sa partie dirigée vers la partie de pied (1b) du support (1) un couteau (27) cylindrique également coaxial au support (1), dont la lame tranchante est constituée par une arête circulaire (58) dirigée vers l'orifice de la partie de pied (1b) du support (1),
— des moyens d'entraînement du porte-couteau (23, 28) par rapport au support, lesdits moyens d'entraînement (8, 25) comportant des moyens d'entraînement du corps (23) à la rotation par rapport au support (1), autour de l'axe (2), et des moyens (22, 24) pour lier à ladite rotation une translation du corps (23) par rapport au support (1) parallèlement à l'axe (2) dans le sens de la formation d'une saillie ou dans le sens d'un escamotage de la lame tranchante (58) par rapport à la partie de pied (1b),

caractérisé en ce que des moyens (17, 20, 21, 63, 65) sont prévus pour entraver ladite translation en autorisant ladite rotation lorsque le corps (23) parvient dans une position prédéterminée par rapport au support (1), lors de ladite translation dans le sens de la formation d'une saillie du couteau hors du support.

2. Trépan selon la revendication 1, caractérisé en ce qu'il comporte des moyens (37) pour régler à volonté ladite position prédéterminée.

3. Trépan selon l'une quelconque des revendications 1 et 2, caractérisé en ce que lesdits moyens d'entraînement (8, 25) sont en prise directement avec le corps (23), en ce que le corps (23) est pourvu à sa surface extérieure d'un filetage (24) coopérant avec un taraudage intérieur (22) porté par le support (1), en ce que ledit taraudage intérieur (22) est porté par un élément (17) mobile en rotation autour de l'axe (2) dans le support (1), entre deux butées (20, 21) écartées angulairement d'un angle prédéterminé, en ce que des moyens sont prévus entre le corps (23) et ledit élément (17) d'une part, et entre ledit élément (17) et le support (1) d'autre part, pour que les forces de frottement tendant à s'opposer à la rotation du porte-couteau (23) par rapport audit élément (17) soient différentes de forces de frottement tendant à s'opposer à la rotation dudit élément (17) par rapport au support (1), en ce que le support (1) et le porte-couteau (23, 28) sont chacun équipées d'un première butée (23a, 45a), les premières butées (45a, 23a) constituant lors de leur venue en butée mutuelle une limite à la rétraction du porte-couteau (23, 28) dans le support (1), en ce que le porte-couteau (23, 28) et ledit élément (17) sont chacun équipés d'une seconde butée (17b, 37a), les secondes butées (17b, 37a) constituant lors de leur contact mutuel une limite au mouvement du porte-couteau (23, 28) par rapport au support (1) en direction de l'oeil, cette limite correspondant à la profondeur de l'incision à réaliser.

4. Trépan selon la revendication 2, caractérisé en ce que lesdits moyens prévus entre le support (1) et ledit élément (17) comportent des butées axiales lisses ou à éléments de roulement (18, 19).

5. Trépan selon l'une quelconque des revendications 3 et 4, caractérisé en ce que lesdits moyens prévus entre le porte-couteau (23, 28) et ledit élément (17) comportent un frein.

6. Trépan selon l'une quelconque des revendications 3 à 5, caractérisé en ce que lesdites deux butées (20, 21) sont constituées par une saillie extérieure (20) dudit élément (17) et par une saillie intérieure (21) du support (1).

7. Trépan selon l'une quelconque des revendications 1 et 2, caractérisé en ce que lesdits moyens d'entraînement (8, 25) sont en prise directement avec le corps (23), en ce que le corps (23) est pourvu à sa surface extérieure d'un filetage (24) coopérant avec un taraudage intérieur (22) porté par le support (1), en ce que ledit taraudage intérieur (22) est porté par un élément (17) mobile autour de l'axe (2) dans le support (1), en ce que des moyens sont prévus entre le corps (23) et ledit élément (17) d'une part, et entre ledit élément (17) et le support (1) d'autre part, pour que les forces de frottement tendant à s'opposer à la rotation du porte-couteau (28) par rapport audit élément (17) soient inférieures aux forces de frottement tendant à s'opposer à la rotation dudit élément (17) par rapport au support (1), et que ledit élément soit néanmoins susceptible d'une rotation par rapport au support lors d'un entraînement du corps (23) à la rotation dans le sens de la formation d'une saillie, mais soit immobilisé par rapport au support (1) lors d'un entraînement du corps (23) à la rotation en sens inverse, en ce que le support (1) et le porte-couteau (23, 28) sont chacun équipés d'une première butée (23a, 45a), les premières butées (45a, 23a) constituant lors de leur venue en butée mutuelle une limite à la rétraction du porte-couteau (23, 28) dans le support (1), en ce que le porte-couteau (23, 28) et ledit élément (17) sont chacun équipés de secondes butées (17b, 37a), les secondes butées (17b, 37a) constituant lors de leur contact mutuel une limite au mouvement du porte-couteau (23, 28) par rapport au support (1) en direction de l'oeil, cette limite correspondant à la profondeur de l'incision à réaliser.

8. Trépan selon la revendication 7, caractérisé en ce que l'élément (17) et le support (1) présentent en regard l'un de l'autre respectivement une face extérieure (17a) et une face intérieure (1c) de révolution autour de l'axe (2), en ce qu'une bague intermédiaire (63) est intercalée entre lesdites faces, à rotation libre vis-à-vis de la première et à rotation avec frottement vis-à-vis de la seconde, les forces de frottement s'opposant à cette rotation étant supérieures aux forces de frottement s'opposant à la rotation du porte-couteau (23, 28) par rapport à l'élément (17), en ce que ladite face extérieure (17a) présente une pluralité d'encoches (66, 66a, 66b) en regard de chacune desquelles la bague intermédiaire (63) présente un perçage traversant radial respectif (64, 64a, 64b), et en ce que dans chaque encoche (66, 66a, 66b) et dans le perçage correspondant est engagée une bille libre (65, 65a, 65b), les formes et dimensions de chaque encoche étant telles que celle-ci constitue une came qui, lors de l'entraînement du porte-couteau (23, 28) à la rotation en sens inverse d'un sens (67) correspondant à la formation d'une saillie, pousse les billes (65, 65a, 65b) dans le sens centrifuge, dans les perçages (64, 64a, 64b) et les applique sous pression contre ladite face intérieure (1c) au support (1) et, lors de l'entraînement du porte-couteau (23, 28) à la rotation dans ledit sens (67) correspondant à la formation d'une saillie, libère les billes (65, 65a, 65b) dans le sens centripète par rapport à ladite face intérieure (1c) du support (1).

9. Trépan selon l'une quelconque des revendications 3 à 8, caractérisé en ce que les premières butées susdites (45a, 23a) limitant la rétraction du porte-couteau dans le corps sont situées de manière que, lorsqu'elles coopèrent, l'arête tranchante (58) du couteau circulaire (27) soit située en retrait par rapport à la face antérieure (4) du support (1).

10. Trépan selon l'une quelconque des revendications 3 à 9, caractérisé en ce que les premières butées (45a, 23a) sont portées de manière fixe par le support (1) et le porte-couteau (23, 28), l'une (17b) des secondes butées est portée de manière fixe par une première des pièces (élément (17) ou porte-couteau (23, 28)) susdites tandis que l'autre (37a) des secondes butées est portée de manière réglable par l'autre pièce, pour constituer lors de leur contact mutuel une limite réglable au mouvement du porte-couteau (23, 28) par rapport au support (1) en direction de l'oeil, cette limite correspondant à la profondeur de l'incision à réaliser, un dispositif (40, 44) d'affichage de la position de ladite butée (37a) par rapport à ladite autre pièce, constitué par un index (44) et une graduation (43) exprimée en unités de profondeur d'incision, étant associé auxdites autre pièce et butée réglable (37a).

11. Trépan selon la revendication 10, caractérisé en ce que l'autre pièce susdite est le porte-couteau (23, 28), pourvu d'un second filetage (38) et d'un couronne crantée (40) qui lui est solidaire, tandis que la butée réglable est constituée par un épaulement (37a) d'une douille filetée (37) coopérant avec ledit second filetage (38), pourvue d'un cliquet élastique (39) coopérant avec ladite couronne crantée (40).

12. Trépan selon la revendication 11, caractérisé en ce que la graduation (43) du dispositif d'affichage susdit (40, 44) est portée par les saillies (42) de la couronne crantée (40) tandis que l'extrémité du cliquet susdit (49) définit, avec l'extrémité d'un masque solidaire de la douille (37), une fenêtre de lecture (44) de ladite graduation (43) constituant ledit index.

13. Trépan selon l'une quelconque des revendications 11 et 12, caractérisé en ce que la partie de pied (1b) du support (1) susdit est démontable pour découvrir ladite douille filetée et constitue une butée extérieure radiale de maintien du cliquet dans sa position.

14. Trépan selon l'une quelconque des revendications 1 à 12, caractérisé en ce que la partie de pied (1b) du support (1) susdit est démontable.

15. Trépan selon l'une quelconque des revendications précédentes, caractérisé en ce que le support (1) est pourvu d'une paroi fixe (45) intérieure au corps (23) du porte-couteau (23, 28) et formant élément de fixation pour une lentille amovible (48, 49).

16. Trépan selon la revendication 15, caractérisé en ce que la lentille (48, 49) susdite possède une face antérieure concave (48a, 49b) complétant la surface antérieure (4) du pied (1b) du support (1) et correspondant à la courbure de l'oeil à traiter.

17. Trépan selon la revendication 16, caractérisé en ce que la lentille susdite (49) est intérieurement évidée (50).

18. Trépan selon l'une quelconque des revendications 15 à 17, caractérisé en ce que la lentille (49) possède un repère de centrage (49a).

19. Trépan selon l'une quelconque des revendications 15 à 18, caractérisé en ce que le support (1) porte, autour du couteau (27), une lentille annulaire (61) en regard de laquelle ladite lentille (48) présente une deuxième face antérieure (48d) disposée en retrait par rapport à ladite face antérieure (48b), et en ce que des moyens sont prévus sur le couteau et/ou le porte-couteau (23, 28) pour autoriser la vision de la lentille annulaire (61) à travers la deuxième face antérieure (48d) de l'autre lentille (48).

20. Trépan selon l'une quelconque des revendications précédentes, caractérisé en ce que le couteau cylindrique (27) est fixé de manière démontable à la partie antérieure du corps (23) du porte-couteau (23, 28), au moyen d'un écrou (28) de fixation et de centrage de préférence attelé de manière imperdable audit corps (23).

21. Trépan selon l'une quelconque des revendications précédentes, caractérisé en ce que lesdits moyens d'entraînement (8, 25) comportent un pignon (8) monté à la rotation dans le support (1) et engrenant avec une couronne dentée (25) solidaire du corps, et des moyens (9) pour animer à volonté ledit pignon (8) d'un mouvement de rotation.

22. Trépan selon la revendication 21, caractérisé en ce que le pignon d'entraînement susdit (8) est en liaison fonctionnelle avec une tige de manoeuvre (7a) pourvue d'un dispositif (9a) de verrouillage de sa rotation et donc d'immobilisation du porte-couteau.

23. Trépan selon l'une quelconque des revendications précédentes, caractérisé en ce que la face antérieure (4) de la partie de pied (1b) dudit support (1) est pourvue de griffes (5') écartées circonférentiellement d'une distance correspondant à l'écartement des points de suture à réaliser.

24. Trépan selon la revendication 23, caractérisé en ce que lesdites griffes (5') sont rétractables.

25. Trépan selon l'une quelconque des revendications précédentes, caractérisé en ce que la face antérieure de la partie de pied du support est pourvue de griffes (5) aptes à empêcher son glissement sur l'oeil sans marquer celui-ci.

26. Trépan selon l'une quelconque des revendications précédentes, caractérisé en ce que le support (1) est équipé de deux embouts (51) débouchant dans l'espace compris entre le support (1) et le porte-couteau (23, 28), reliant cet espace à une source de fluide et à une source d'aspiration.

27. Trépan selon l'une quelconque des revendications précédentes, caractérisé en ce que le couteau (27) présente deux arêtes inférieures circulaires (58) concentriques, occupant des positions relatives prédéterminées, dont chacune définit une lame tranchante.

## Patentansprüche

1. Trepan zur Ausführung mindestens eines kreisförmigen Einschnittes an der Augenhornhaut, mit:

— einem äußeren, rohrförmigen, eine Umhüllung bildenden Halter (1), der eine Achse (2) festlegt und einen Fußteil (1b) zum Aufsetzen auf das Auge aufweist,
— einem im Halter beweglichen inneren Messerträger (23, 28), der einen zum Halter (1) koaxialen Körper (23) aufweist und an seinem gegen den Fußteil (1b) des Halters (1) gerichteten Teil ein zylindrisches, gleichfalls zum Halter (1) koaxiales Messer (27) aufweist, dessen Schneide durch eine gegen die Öffnung des Fußteiles (1b) des Halters (1) gerichtete kreisförmigen Kante (58) gebildet ist,
— Mittel zum Antrieb des Messerträger (23, 28) in bezug auf den Halter, welche Antriebsmittel (8, 25) Mittel zum Drehen des Körpers (23) relativ zum Halter (1) um die Achse (2) und Mittel (22, 24) aufweisen, durch die mit der genannten Drehung eine geradlinige Bewegung des Körpers (23) in bezug auf den Halter (1) parallel zur Achse (2) verbunden wird, um ein Vorspringen oder Zurückziehen der Schneide (58) in bezug auf den Fußteil (1b) zu bewirken,

dadurch gekennzeichnet, daß Mittel (17, 20, 21, 63, 65) vorgesehen sind, um die genannte geradlinige Bewegung unter Zulassung der genannten Drehung zu behindern, wenn der Körper (23) bei der genannten geradlinigen Bewegung im Sinne eines Vorspringens des Messers über den Halter in einer vorherbestimmten Position in bezug auf den Halter (1) anlangt.

2. Trepan nach Anspruch 1, dadurch gekennzeichnet, daß er Mittel (37) zum beliebigen Einstellen der genannten vorherbestimmten Position aufweist.

3. Trepan nach einem der Ansprüche 1 und 2, dadurch gekennzeichnet, daß die genannten Antriebsmittel (8, 25) in direktem Eingriff mit dem Körper (23) stehen, daß der Körper (23) an seiner Außenfläche mit einem Gewinde (24) versehen ist, das mit einem vom Halter (1) getragenen Innengewinde (22) zusammenarbeitet, daß das genannte Innengewinde (22) von einem um die Achse (2) im Halter (1) zwischen zwei winkelmäßig um einen vorherbestimmten Winkel gegeneinander versetzen Anschlägen (20, 21) drehbeweglichen Element (17) getragen ist, daß zwischen dem Körper (23) und dem genannten Element (17) einerseits und zwischen dem genannten Element (17) und dem Halter (1) andererseits Mittel vorgesehen sind, um die Reibungskräfte, die sich der Drehung des Messerträgers (23) in bezug auf das genannte Element (17) entgegensetzen, verschieden von den Reibungskräften zu halten, die sich der Drehung des genannten Elementes (17) in bezug auf den Halter (1) entgegensetzen, daß der Halter (1) und der Messerträger (23, 28) je mit einem ersten Anschlag (23a, 45a) versehen sind, welche ersten Anschläge (45a, 23a) bei ihrem gegenseitigen Kontakt eine Begrenzung für die Zurückziehung des Messerträgers (23, 28) im Halter (1) bilden, daß der Messerträger (23, 28) und das genannte Element (17) je mit einem zweiten Anschlag (17b, 37a) versehen sind, welche zweiten Anschläge (17b, 37a) bei ihrem gegenseitigen Kontakt eine Begrenzung für die Bewegung des Messerträgers (23, 28) in Richtung auf das Auge in bezug auf den Halter (1) darstellen, welche Begrenzung der Tiefe des auszuführenden Einschnittes entspricht.

4. Trepan nach Anspruch 3, dadurch gekennzeichnet, daß die genannten, zwischen dem Halter (1) und dem genannten Element (17) vorgesehenen Mittel glatte Axialanschläge oder Axialanschläge mit Rollelementen (18, 19) aufweisen.

5. Trepan nach einem der Ansprüche 3 und 4, dadurch gekennzeichnet, daß die genannten, zwischen dem Messerträger (23, 28) und dem genannten Element (17) vorgesehenen Mittel eine Bremse aufweisen.

6. Trepan nach einem der Ansprüche 3 bis 5, dadurch gekennzeichnet, daß die genannten beiden Anschläge (20, 21) durch einen äußeren Vorsprung (20) des genannten Elementes (17) und einen inneren Vorsprung (21) des Halters (1) gebildet sind.

7. Trepan nach einem der Ansprüche 1 und 2, dadurch gekennzeichnet, daß die genannten Antriebsmittel (8, 25) in direktem Eingriff mit dem Körper (23) an seiner Außenfläche mit einem Gewinde (24) versehen ist, das mit einem durch den Halter (1) getragenen Inenngewinde (22) zusammenwirkt, daß das genannte Innengewinde (22) von einem um die Achse (2) im Halter (1) bewegbaren Element (17) getragen ist, daß zwischen dem Körper (23) und dem genannten Element (17) einerseits und dem genannten Element (17) und dem Halter (1) anderseits Mittel vorgesehen sind, um die Reibungskräfte, die sich der Drehung des Messerträgers (28) in bezug auf das genannte Element (17) entgegensetzen, niedriger als die Reibungskräfte zu halten, die sich der Drehung des genannten Elementes (17) in bezug auf den Halter (1) entgegensetzen, wobei sich das genannte Element bei einer Drehung des Körpers (23) im Sinn der Bildung eines Vorsprunges trotzdem in bezug auf den Halter drehen kann, bei einer Drehung des Körpers (23) im umgekehrten Drehsinn jedoch in bezug auf den Halter (1) unbeweglich gehalten wird, daß der Halter (1) und der Messerträger (23, 28) je mit einem ersten Anschlag (23a, 45a) versehen sind, welche ersten Anschläge (45a, 23a) bei ihrem gegenseitigen Kontakt eine Begrenzung für die Zurückziehung des Messerträgers (23, 28) im Halter (1) bilden, daß der Messerträger (23, 28) und das genannte Element (17) je mit einem zweiten Anschlag (17b, 37a) versehen sind, welche zweiten Anschläge (17b, 37a) bei ihrem gegenseitigen Kontakt eine Begrenzung für die Bewegung des Messerträgers (23, 28) in Richtung auf das Auge in bezug auf den Halter (1) darstellen, welche Begrenzung der Tiefe des auszuführenden Einschnittes entspricht.

8. Trepan nach Anspruch 7, dadurch gekennzeichnet, daß das Element (17) und der Halter (1) ihnen jeweils gegenüberliegend um die Achse (2) umlaufende Außen- (17a) bzw. Innenflächen (1c) aufweisen, daß ein Zwischenring (63) zwischen den genannten Flächen mit freier Drehung gegenüber der ersten Fläche und Reibungsdrehung gegenüber der zweiten Fläche eingelegt ist, wobei die sich dieser Drehung entgegensetzenden Reibungskräfte größer sind als die Reibungskräfte, die sich der Drehung des Messerträgers (23, 28) in bezug auf das Element (17) entgegensetzen, daß die genannte Außenfläche (17a) eine Mehrzahl von Kerben (66, 66a, 66b) aufweist, denen gegenüber der Zwischenring (63) jeweils eine entsprechende radial durchgehende Bohrung (64, 64a, 64b) aufweist, und daß in jeder Kerbe (66, 66a, 66b) und der entsprechenden Bohrung eine freie Kugel (65, 65a, 65b) vorgesehen ist, wobei die Formen und Abmessungen jeder Kerbe so gewählt sind, daß sie Nocken bilden, die bei Drehung des Messerträgers (23, 28) im umgekehrten Drehsinn zu dem Drehsinn (67), der der Bildung eines Vorsprunges entspricht, die Kugeln (65, 65a, 65b) in Zentrifugalrichtung in die Bohrungen (64, 64a, 64b) drükken und sie unter Druck gegen die genannte Innenfläche (1c) am Halter (1) anlegen und bei Drehung des Messerträgers (23, 28) im genannten, der Bildung eines Vorsprunges entsprechenden Drehsinn (67) die Kugeln (65, 65a, 65b) in Zentripetalrichtung in bezug auf die genannte Innenfläche (1c) des Halters (1) freigeben.

9. Trepan nach einem der Ansprüche 3 bis 8, dadurch gekennzeichnet, daß die genannten ersten, das Zurückziehen des Messerträgers in den Körper begrenzenden Anschläge (45a, 23a) so angeordnet sind, daß bei ihrem Zusammenarbeiten die Schneide (58) des kreisförmigen Messers (27) bezüglich der vorderen Fläche (4) des Halters (1) zurückgezogen liegt.

10. Trepan nach einem der Ansprüche 3 bis 9, dadurch gekennzeichnet, daß die ersten Anschläge (45a, 23a) fest auf dem Halter (1) und dem Messerträger (23, 28) angeordnet sind, und einer (17b) der zweiten Anschläge fest an einem ersten der genannten Teile (Element (17) oder Messerträger (23, 28)) angeordnet ist, während der anderen (37a) der zweiten Anschläge verstellbar auf dem anderen Teil angeordnet ist, um bei ihrem gegenseitigen Kontakt eine einstellbare Begrenzung für die Bewegung des Messerträgers (23, 28) in Richtung auf das Auge in bezug auf den Halter (1) zu bilden, welche Begrenzung der Tiefe des auszuführenden Einschnittes entspricht, wobei eine durch einen Index (44) und eine in Einschnittiefeneinheiten ausgedrückte Skala (43) gebildete Einrichtung (40, 44) zur Anzeige der Position des genannten Anschlages (37a) in bezug auf den genannten anderen Teil dem genannten anderen Teil und verstellbaren Anschlag (37a) zugeordnet ist.

11. Trepan nach Anspruch 10, dadurch gekennzeichnet, daß der andere vorgenannte Teil der mit einem zweiten Gewinde (38) und einem mit ihm fest verbundenen Zahnkranz (40) versehene Messerträger (23, 28) ist, während der verstellbare Anschlag durch einen Bund (37a) einer mit dem genannten zweiten Gewinde (38) zusammenwirkenden und eine mit dem genannten Zahnkranz (40) zusammenwirkende elastische Klinke (39) aufweisenden Gewindehülse (37) gebildet ist.

12. Trepan nach Anspruch 11, dadurch gekennzeichnet, daß die Skala (43) der vorgenannten Anzeigeeinrichtung (40, 44) von den Vorsprüngen (42) des Zahnkranzes (40) getragen wird, während das Ende der genannten Klinke (49) mit dem Ende einer mit der Hülse (37) fest verbundenen Maske ein den genannten Index bildendes Ablesefenster (44) für die genannte Skala (43) definiert.

13. Trepan nach einem der Ansprüche 11 und 12, dadurch gekennzeichnet, daß der Fußteil (1b) des genannten Halters (1) zum Freilegen der genannten Gewindehülse abnehmbar ist und einen äußeren radialen Anschlag zum Festhalten der Klinke in ihrer Position bildet.

14. Trepan nach einem der Ansprüche 1 bis 12, dadurch gekennzeichnet, daß der Fußteil (1b) des Halters (1) abnehmbar ist.

15. Trepan nach einem der vorhergehenden Ansprüche, dadurch gekennzeichnet, daß der Halter (1) mit einer festen Wand (45) im Inneren des Körpers (23) des Messerträgers (23, 28) versehen ist, die als Befestigungselement für eine abnehmbare Linse (48, 49) dient.

16. Trepan nach Anspruch 15, dadurch gekennzeichnet, daß die genannte Linse (48, 49) eine vordere konkave Fläche (48a, 49b) aufweist, die die vordere Fläche (4) des Fußteiles (1b) des Halters (1) komplettiert und der Krümmung des zu behandelnden Auges entspricht.

17. Trepan nach Anspruch 16, dadurch gekennzeichnet, daß die genannte Linse (49) innen hohl ist (50).

18. Trepan nach einem der Ansprüche 15 bis 17, dadurch gekennzeichnet, daß die Linse (49) eine Zentriermarke (49a) aufweist.

19. Trepan nach einem der Ansprüche 15 bis 18, dadurch gekennzeichnet, daß der Halter (1) rund um das Messer (27) eine ringförmige Linse (61) trägt, der gegenüber die genannte Linse (48) eine zweite vordere, in bezug auf die genannte vordere Fläche (48b) zurückversetzt angeordnete Fläche (48d) aufweist, und daß auf dem Messer und/oder dem Messerträger (23, 28) Mittel vorgesehen sind, um ein Sehen der ringförmigen Linse (61) durch die zweite vordere Fläche (48d) der anderen Linse (48) zu erlauben.

20. Trepan nach einem der vorhergehenden Ansprüche, dadurch gekennzeichnet, daß das zylinderförmige Messer (27) mittels einer vorzugsweise unverlierbar mit dem genannten Körper (23) verbundenen Fixier- und Zentriermutter (28) abnehmbar am vorderen Teil des Körpers (23) des Messerträgers (23, 28) befestigt ist.

21. Trepan nach einem der vorhergehenden Ansprüche, dadurch gekennzeichnet, daß die genannten Antriebseinrichtungen (8, 25) ein drehbar im Halter (1) befestigtes und in einen mit dem Körper kraftschlüssig verbundenen Zahnkranz (25) eingreifendes Antriebsrad (8) aufweist, sowie Einrichtungen (9), um das genannte Antriebsrad (8) beliebig in eine Drehbewegung zu versetzen.

22. Trepan nach Anspruch 21, dadurch gekennzeichnet, daß das genannte Antriebsrad (8) in funktioneller Verbindung mit einer Betätigungsstange (7a) steht, die mit einer Vorrichtung (9a) zur Verriegelung seiner Drehung und damit zum Unbeweglichmachen des Messerträgers versehen ist.

23. Trepan nach einem der vorhergehenden Ansprüche, dadurch gekennzeichnet, daß die vordere Fläche (4) des Fußteiles (1b) genannten Halters (1) mit Klauen (5') versehen ist, die über den Umfang in Abständen verteilt sind, die den Abständen der anzubringenden Nahtpunkte entsprechen.

24. Trepan nach Anspruch 23, dadurch gekennzeichnet, daß die genannten Klauen (5') einziehbar sind.

25. Trepan nach einem der vorhergehenden Ansprüche, dadurch gekennzeichnet, daß die vordere Fläche des Fußteiles des Halters mit Klauen (5) versehen ist, die sein Gleiten auf dem Auge ermöglichen, ohne das Auge zu markieren.

26. Trepan nach einem der vorhergehenden Ansprüche, dadurch gekennzeichnet, daß der Halter (1) mit zwei Aushöhlungen (51) versehen ist, die in den Raum zwischen dem Halter (1) und dem Messerträger (23, 28) münden und diesen Raum mit einer Flüssigkristallquelle und einer Luftansaugquelle verbinden.

27. Trepan nach einem der vorhergehenden Ansprüche, dadurch gekennzeichnet, daß das Messer (27) zwei untere kreisförmige konzentrische Kanten (58) aufweist, die vorbestimmte relative Positionen einnehmen und deren jede eine Schneide darstellt.

## Claims

1. A trepan intended to make at least one circular incision on the cornea of the eye, comprising

— an exterior tubular support (1) forming a casing, having an axis (2) and possessing a foot piece (1b) for application on the eye,
— an interior knife holder capable of moving in the support, the knife holder (23, 28) having a body (23) coaxial to the support (1) and carrying in the part directed towards the foot piece (1b) of the support (1) a cylindrical knife (27), likewise coaxial to the support (1), of which the cutting blade consists of a circular edge (58) directed towards the aperture of the foot piecer (1b) of the support (1),
— means for driving the knife holder (23, 28) in relation to the support, the said driving means (8, 25) having driving means for the rotation of the body (23) in relation to the support (1) around the axis (2), and devices (22, 24) for linking to the said rotation a translation of the body (23), in relation to the support (1), parallel to the axis (2) in the direction which a projection is formed or in the direction of a retraction of the cutting blade (58) in relation to the foot piece (1b),

characterised in that means (17, 20, 21, 63, 65) are provided to obstruct the said translation while allowing the said rotation when the body (23) reaches a predetermined position in relation to the support (1), in the case of translation in the direction in which the knife is caused to project from the support.

2. A trepan according to claim 1, characterised in that it comprises devices (37) for adjusting the said predetermined position as required.

3. A trepan according to one of claims 1 and 2, characterised in that the said driving means (8, 25) engage directly with the body (23), in that the body (23) is provided on its exterior surface with a screw thread (24) in co-operation with an internal screw thread (22), carried by the support (1), in that the said interior screw thread (22) is carried by an element (17) which is rotatable around the axis (2) in the support (1), between two stop members (20, 21) offset angularly at a predetermined angle, in that means are provided between the body (23) and the said element (17) on the one hand and between the said element (17) and the support (1), on the other hand so that the friction forces, tending to oppose the rotation of the knife holder (23) in relation to the said element (17), are different from the friction forces tending to oppose the rotation of the said element (17) in relation to the support (1), in that the support (1) and the knife-holder (23, 28) are each equipped with a first stop member (23a, 45a), the first stop members (45a, 23a) constituting, when they abut against each other, a limit to the retraction of the knife holder (23, 28) in the support (1), in that the knife holder (23, 28) and the said element (17) are each equipped with a second stop member (17b, 37a), the second stop members (17b, 37a) constituting on mutual contact, a limit to the movement of the knife holder (23, 28) in relation to the support (1) in the direction of the eye, this limit corresponding to the depth of the incision to be made.

4. A trepan according to claim 3, characterised in that the said means provided between the support (1) and the said element (17) comprise axial stop members which are smooth or have bearing elements (18, 19).

5. A trepan according to one of claims 3 and 4, characterised in that the said means provided between the knife holder (23, 28) and the said element (17) comprise a brake.

6. A trepan according to one of claims 3 to 5, characterised in that the said two stop members (20, 21) consist of an exterior projection (20) of the said element (17) and of in interior projection (21) of the support (1).

7. A trepan according to one of claims 1 and 2, charaterised in that the said driving means (8, 25) engage directly with the body (23), in that the body (23) is provided on its exterior surface with a screw thread (24) in co-operation with an interior screw thread (22), carried by the support (1), in that the said interior screw thread (22) is carried by an element (17) capable or moving around the axis (2) in the support (1), in that devices are provided between the body (23) and the said element (17) on the one hand, and between the said element (17) and the support (1), on the other hand, so that the friction forces tending to oppose the rotation of the knife holder (28) in relation to the said element (17) are lower than the friction forces tending to oppose the rotation of the said element (17) in relation to the support (1), and that the said element in nevertheless capable of rotating in relation to the support, in the case of the body (23) being driven in rotation in the direction in which a projection is formed, but is fixed securely in relation to the support (1) in the case of the body (23) being driven in rotation in the opposite direction, in that the support (1) and the knife holder (23, 28) are each equipped with a first stop member (23a, 45a), the first stop members (45a, 23a) constituting, when they abut against each other, a limit to the retraction of the knife holder (23, 28) in the support (1), in that the knife holder (23, 28) and the said element (17) are each equipped with second stop members (17b, 37a), then second stop members (17b, 37a) constituting, on mutual contact, a limit to the movement of the knife holder (23, 28) in relation to the support (1) in the direction of the eye, this limit corresponding to the depth of the incision to be made.

8. A trepan according to claim 7, characterised in that the element (17) and the support (1) have, facing each other, respectively an exterior face (17a) and an interior face (1c) of revolution around the axis (2), in that an intermediate ring (63) is inserted between the said faces, in free rotation relative to the first, and in frictional rotation relative to the second, the friction forces opposing this rotation being higher than the friction forces opposing the rotation of the knife holder (23, 28) in relation to the element (17), in that the said exterior face (17a) has several notches (66, 66a, 66b) opposite each one of which the intermediate ring (63) has a respective radial transverse opening (64, 64a, 64b), and in that in each notch (66, 66a, 66b) and in the corresponding opening a free ball is engaged (65, 65a, 65b), the shapes and dimensions of each notch being such that it constitutes a cam which, when the knife holder (23, 28) is driven in rotation in the opposite direction to the direction (67) in which a projection is formed, pushes the balls (65, 65a, 65b) in the centrifugal direction, into the openings (64, 64a, 64b) and applies them under pressure against the said interior face (1c) to the support (1) and, when the knife holder (23, 28) is driven in rotation in the said direction (67) in which a projection is formed, releases the balls (65, 65a, 65b) in the centripetal direction in relation to the said interior face (1c) of this support (1).

9. A trepan according to one of claims 3 to 8, characterised in that the first above-mentioned stop members (45a, 23a) limiting the retraction of the knife holder in the body, are situated in such a manner that when they co-operate, the cutting edge (58) of the circular knife (27) is situated in a retracted position in relation to the front face (4) of the support (1).

10. A trepan according to one of claims 3 to 9, characterised in that the first stop members (45a, 23a) are carried in fixed manner by the support (1) and the knife holder (23, 28), one (17b) of the second stop members is carried in fixed manner by one of the above-mentioned parts (element (17) or knife holder (23, 28)), while the other (37a) second stop member is carried in adjustable manner by the other part to constitute, on mutual contact, an adjustable limit to the movement of the knife holder (23, 28) in relation to the support (1) in the direction of the eye, this limit corresponding to the depth of the incision to be made, a device (40, 44) for displaying the position of the said stop member (37a) in relation to the said other part, consisting of an index (44) and a scale (43) expressed in units of depth of incision, being connected to the said other part and adjustable stop member (37a).

11. A trepan according to claim 10, characterised in that the other above-mentioned part is the knife holder (23, 28), provided with a second screw thread (38) and a notched ring (40) which is integral with it, while the adjustable stop member consists of a shoulder (37a) of a threaded socket (37) co-operating with the said second screw thread (38), provided with an elastic catch (39) co-operating with the said notched ring (40).

12. A trepan according to claim 11, characterised in that the scale (43) of the above-mentioned display device (40, 44) is carried by the projections (42) of the notched ring (40) while the end of the abovementioned catch (49) defines, with the end of a mask integral with the socket (37), a window (44) for reading the said scale (43) constituting the said index.

13. A trepan according to one of claims 11 and 12, characterised in that the foot piece (1b) of the above-mentioned support (1) can be detached to uncover the said threaded socket and constitutes an exterior radial stop member for holding a catch in its position.

14. A trepan according to one fo claims 1 to 12, characterised in that the foot piece (1b) of the above-mentioned support (1) is detachable.

15. A trepan according to one of the preceding claims, characterised in that the support (1) is provided with a fixed wall (45) interior to the body (23) of the knife holder (23, 28) and forming the fixing element for a removable lens (48, 49).

16. A trepan according to claims 15, characterised in that the above-mentioned lens (48, 49) has a concave front face (48a, 49b) completing the front surface (4) of the foot (1b) of the support (1) and corresponding to the curvature of the eye to be treated.

17. A trepan according to claim 16, characterised in that the above-mentioned lens (49) has a hollow interior (50).

18. A trepan according to one of claims 15 to 17, characterised in that the lens (49) has a centering mark (49a).

19. A trepan according to one of claims 15 to 18, characterised in that the support (1) carries, around the knife (27), an annular lens (61) opposite which the said lens (48) has a second front face (48d) positioned in a retracted position in relation to the said front face (48b), and in that means are provided on the knife and/or the knife holder (23, 28) to allow the annular lens (61) to be seen through the second anterior face (48d) of the other lens (48).

20. A trepan according to one of the preceding claims, characterised in that the cylindrical knife (27) is fixed in a detachable manner to the front part of the body (23) of the knife holder (23, 28), by means of a screw nut (28) for fixing and centering, preferably coupled in such a manner that it cannot be lost from the said body (23).

21. A trepan according to one of the preceding claims, characterised in that the said driving means (8, 25) consist of a pinion (8) mounted rotatably in the support (1) and engaging with the toothed ring (25) which is integral with the body, and means (9) for driving the said pinion (8) in a movement of rotation as required.

22. A trepan according to claim 21, characterised in that the above-mentioned driving pinion (8) is in operational linkage with a control rod (7a) provided with a device (9a) for locking the rotation and thus firmly securing the knife holder.

23. A trepan according to one of the preceding claims, characterised in that the front face (4) of the foot piece (1b) of the said support (1) is provided with grips (5') spaced on the circumference at a distance corresponding to the spacing of the sutures to be made.

24. A trepan according to claim 23, characterised in that the said grips (5') are retractable.

25. A trepan according to one of the preceding claims, characterised in that the front face of the foot piece of the support is provided with grips (5) which are suitable for preventing it slipping onto the eye without marking the eye.

26. A trepan according to one of the preceding claims, characterised in that the support (1) is equipped with two joining pieces (51) emerging into the space between the support (1) and the knife holder (23, 28), connecting this space with a fluid source and a suction source.

27. A trepan according to one of the preceding claims, characterised in that the knife (27) has two concentric lower circular edges (58), occupying predetermined relative positions, of which each defines a cutting blade.

FIG_1

FIG_2

FIG_3

FIG_7

FIG_4

FIG_5

FIG_6

0 047 190

FIG_8

FIG_9

FIG_10

FIG_11

FIG_12

FIG_14

FIG_13